(19) Europäisches Patentamt
European Patent Office
Office européen des brevets

(11) Publication number: **0 171 840**
**B1**

(12) **EUROPEAN PATENT SPECIFICATION**

(45) Date of publication of patent specification: **22.06.88**

(21) Application number: **85201146.9**

(22) Date of filing: **09.07.85**

(51) Int. Cl.[4]: **C 07 C 51/00,** C 07 C 57/30, C 07 C 59/64, C 07 C 59/68, C 07 C 67/475, C 07 C 69/734, C 07 D 333/22

(54) **Process for the preparation of arylalkanoic acids by oxidative rearrangement of arylalkanones.**

(30) Priority: **13.07.84 IT 2188384**

(43) Date of publication of application: **19.02.86 Bulletin 86/08**

(45) Publication of the grant of the patent: **22.06.88 Bulletin 88/25**

(84) Designated Contracting States: **AT BE CH DE FR GB IT LI LU NL SE**

(56) References cited:

**JOURNAL OF THE CHEMICAL SOCIETY, PERKIN TRANSACTIONS I, 1982, pages 235-242; S.D. HIGGINS et al.: "Conversion of aromatic ketones into pi-arylalkanoic acids. Oxidation by thallium (III) and by halogens"**

**The file contains technical information submitted after the application was filed and not included in this specification**

(73) Proprietor: **BLASCHIM S.p.A.**
**Via Vittor Pisani, 28**
**I-20124 Milano (IT)**

(72) Inventor: **Citterio, Attilio**
**Via Borgazzi, 4**
**Monza (IT)**
Inventor: **Tinucci, Laura**
**Via Verdi, 4**
**San Giuliano Milanese (MI) (IT)**
Inventor: **Filippini, Lucio**
**Via Fratelli Cervi, 27**
**Saronno (VA) (IT)**
Inventor: **Belli, Aldo**
**Via Quadri, 2**
**Cornate d'Adda (MI) (IT)**

(74) Representative: **Marchi, Massimo et al**
**c/o Marchi & Mittler s.r.l. Viale Lombardia 20**
**I-20131 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 171 840 B1**

Courier Press, Leamington Spa, England.

**Description**

This invention relates to a process for preparing an arylalkanoic acid which comprises the addition of iodine to a mixture of an aryalkanone and an excess of an orthoester, heating of the reaction mixture, the addition of an inorganic base and finally of an acid.

More particularly, this invention relates to the preparation of an arylalkanoic acid according to the following reaction diagram:

$$\underset{(I)}{Ar{-}CO{-}CH_2{-}X} \rightarrow \underset{(II)}{Ar{-}\overset{\overset{\displaystyle X}{|}}{C}H{-}COOH}$$

wherein X is H or a $C_1$—$C_4$ alkyl radical, and
Ar is selected from the group comprising an aryl, a substituted aryl, a fused heterocyclic aryl, a heterocycle, a substituted heterocycle and a fused aryl heterocycle radical.

It is known that many arylalkanoic acids are useful as drugs and others as intermediates. More particularly many members of this class are known to be useful as anti-inflammatory, analgesic, and antipiretic agents. Examples of these compounds include Thiaprofenic acid, Ibuprofen, Fenclorac, Indoprofen, Flurbiprofen, Naproxen, Ketoprofen, Fenoprofen, Piroprofen, Suprofen, Aclofenac, Xenbucin, Diclofenac and Tolmetin (Antiinflammatory Drugs, Springer Verlag, 1979, pages 321—3) and Isoprofen, FLP—58,302 (CAS—58282—60—3—), Furofenac, Cicloprofen, Y—8004 (Drugs of the Future 2, 217 (1977)), Caroprofen, Benoxaprofen, Y—9213 (Drugs of the Future 4, 373, 1978), Enprofen, Benzofenac, Fenclofenac, Isoxepac, Oxepinal, Tiopinac, Zomepirac, and Fentiazac.

Because of the great interest assumed by arylalkanoic acids, in recent years research has been intensified to find a preparation method which would make it possible to manufacture the acids of Formula II starting from unexpensive compounds such as the ketones of Formula I in a single vessel and without isolating and purifying intermediate products if any.

A first attempt is the one described in U.S. Patents Nos. 4,107,439, 4,135,051 and 4,412,054, which require the use of trivalent thallium nitrate. This process suffers from many draw-backs but the main one is that it gives rise to highly toxic by-products which do not allow the use of arylalkanoic acids thus prepared as pharmaceuticals.

S. D. Higgins and C. B. Thomas (J. C. S. Perkin Trans, 1982, 235—42; idem, 1983, 1483—88) put forward various hypotheses about the mechanism of said reaction for the purpose of replacing thallium nitrate with nontoxic and more economical reagents and to check the correctness of their hypotheses, they tested various oxidazing systems; the best yields are given by the system consisting of iodine, silver nitrate, trimethylorthoformate and methanol but the authors themselves acknowledge that the large quantity of silver nitrate required makes this process uneconomical (ibid, 1982, 239) and that the reaction does not proceed at all in the absence of silver nitrate (ibid, 1983, 1483).

In European Patent Appliction No. 108.442 a method is described according to which the silver nitrate is substituted by zinc chloride but the yields obtained with this method are rather small.

It has now been found that the system described by S. D. Higgins et al. affords excellent yields even in the absence of silver nitrate and methanol provided a substantial excess of alkylorthoformate or other orthoesters is used.

This invention relates to a process for preparing an arylalkanoic acid of Formula II which comprises the addition of iodine to a mixture of an arylalkanone of Formula (I) and an excess of an orthoester, heating of the mixture thus obtained, the addition of an inorganic base, and finally the addition of an acid.

To the reaction mixture may be added a suitable solvent, a diluent and/or a catalytic quantity of a protic acid.

The iodine is added in the amount of approximately 1.05 mole for each mole of arylalkanone. The quantity of iodine used may be substantially reduced by adding a suitable oxidant which restores the iodine from the hydriodic acid which is formed in the course of the reaction. It has been also noted that the presence of a peroxide plays an important role in the kinetics of the process which, for the more reactive systems, is expressed with a considerable reduction in reaction times while in less reactive systems or those which interact with hydriodic acid it is expressed by appreciable increases in yields. Examples of suitable oxidants are hydrogen peroxide, the organic peroxides, preferably the diacylperoxides such as dibenzoyl or dodecanoyl peroxide, the peracids such as m-chloroperbenzoic and permaleic acid, the peresters such as tert-butyl-peracetate, tert-butylperbenzoate, di-tert-butylperoxalate, di-tert-butylperisobutyrate, and di-tert-butyl-cyclohexylpercarbonate and the hydroperoxides such as, for example, tert-butylhydroperoxide and cumylhydroperoxide.

The orthoester is added in the amount of at least 2 mole for each mole of arylalkanone; the addition of quantities between 1 and 2 mole gives smaller yields while the addition of more than 10 mole does not give appreciable advantages.

Suitable orthoesters are the alkylorthoformates, the alkylorthoacetates and the alkylorthocarbonates in which the alkyl has from 1 to 10 carbon atoms and preferably from 1 to 4 carbon atoms.

The presence of a solvent, a diluent and/or a catalytic quantity of a protic acid is not strictly necessary.

The addition of a solvent or a diluent is nevertheless useful when the arylalkanone is not very soluble in the orthoester or when it is not very reactive at the boiling temperature of the orthoester.

Examples of solvent which increase the solubility of the arylalkanone in the reaction mixture are the aliphatic alcohols and glycols with 1—10 carbon atoms. Selection of the aliphatic alcohol and orthoester will preferably be made in such a way that the alcohol corresponds to that used as a precursor of the orthoester. When a glycol is used it is preferable to operate in the presence of the corresponding orthocarbonate.

When the arylalkanone reacts slowly at the boiling temperature of the orthoester it will be preferable to add an inert organic diluent having a high boiling point such as for example benzene, toluene, xylene and nitrobenzene.

The addition of a protic acid is also useful to bring the arylalkanone into solution.

Examples of suitable protic acids are hydrochloric, sulfuric, p-toluenesulfonic and methanesulfonic acid.

According to this invention a mixture of an arylalkanone and an orthoester and, optionally, a solvent, a diluent and/or a protic acid is refluxed until it becomes clear. This occurs in between 5 minutes and 24 hours. Iodine is then added at a temperature comprised between room temperature and the boiling temperature of the reaction mixture and, when the quantity of iodine is less than 1.05 mole for each mole of arylalkanone, an oxidant is also added.

The reaction mixture is then again heated to reflux until a sample of the reaction mixture does not reveal the presence of the keto group when treated with an aqueous solution of 5% hydrochloric acid. The time required for the keto group to disappear varies with the reactivity of the arylalkanone and the boiling temperature of the reaction mixture and can thus vary between one-half hour and 170 hours.

When the reaction is over the excess iodine is destroyed by adding a reducing agent such as sodium sulfite, sodium hydrosulfite and sodium thiosulfate and an inorganic base is added.

Examples of suitable inorganic bases are sodium hydroxide and potassium hydroxide.

The mixture thus obtained is again heated to boiling for from 30 minutes to six hours. Water is added, the mixture is acidified and the desired arylalkanoic is isolated with high yields. Said isolation is performed with the usual techniques such as filtration, extraction with solvents, or fractional distillation.

The above process is carried out in a single vessel without isolating any intermediate product.

The iodine may be recovered in the form of alkyl iodide by distillation or in the form of iodine by oxidation of the mother liquors, for example with chlorine or sodium hypochlorite.

The solvents and/or the diluents are also easily recovered by fractionated distillation.

The process according to this invention thus makes it possible to prepare the arylalkanoic acids of Formula II in an extremely economical manner because high yields are obtained, because economical or in any case readily recovered raw materials are use, and because the process is carried out in a single vessel with no particular employment of labour.

The following examples illustrate the invention without limiting it.

### Example 1

Preparation of dl 2-(6'-methoxy-2'-naphthyl)-propionic acid:

a) Iodine (136 g; 0.53 mole) was added to a solution of 1-(6'-methoxy-2'-naphthyl)-1-propanone (100 g; 0.47 mole), toluene (120 ml), methanol (4 g), and trimethylorthoformate (110 g; 1.04 mole) at 16°C. After 10 minutes the solution was heated to 26°C and kept at this temperature for 1 hour. Trimethylorthoformate (100 g; 0.98 mole) was added and the mixture was heated to reflux (476°C) for 22 hours.

The mixture was cooled and sodium sulfite (10 g), sodium hydroxide drops (20 g; 0.5 mole), and water (20 ml) were added and the mixture was heated to 60°C for two hours. 5% hydrochloric acid (0.36 l) was added and the layers were decanted. The organic layer was dried and the solvent was removed by distillation.

Yield, 90%; m.p. 154—155°C.

Similar resutls were obtained by substituting:

—triethylorthoformate for the trimethylorthoformate (yield, 80%); and

— nitrobenzene for the toluene (yield, 82%).

b) To a solution of trimethylorthoformate (14.4 ml; 0.14 mole) in methanol (40 ml; 0.99 mole) was added 0.1 ml of a 48% solution of hydrochloric in methanol and the 1-(6'-methoxy-2'-naphthyl)-1-propanone (10 g; 0.047 mole). After 10 minutes the solution became homogeneous and was maintained under reflux for 2 hours. It was then cooled to 40°C and iodine (11.85 g; 0.047 mole) was added. The reaction mixture was refluxed for 30 hours, the solvent was removed by distillation and the residue was treated with potassium hydroxide in methanol (0.20 mole in 40 ml) at boiling for 2 hours. The reaction mixture was evaporated to dryness, dissolved in water and extracted with ethyl ether. The aqueous layer was acidified to precipitate the dl 2-(6'-methoxy-2'-naphthyl)-propionic acid weighing after drying 8.65 g; m.p. 154—155°C (yield, 80%).

Similarly:

1-(6'-methoxy-5'-bromo-2'-naphthyl)-1-propanone (10 g; 0.034 mole) in methanol (34 ml), trimethylorthoformate (11.2 ml; 0.102 mole) and iodine (8.66 g) were preheated to reflux for 72 hours. Then

the mixture was treated in a manner similar to that described above, 80% of dl 2-(6'methoxy-5'-bromo-2'-naphthyl)-1-propionic acid.

4-methoxy-propiophenone (5 g; 0.03 mole) in methanol (25 ml), trimethylorthoformate (10 ml; 0.09 mole), and iodine (7.62 g); 0.03 mole) were heated to reflux for 30 hours. Then the reaction mixture was treated in a manner similar to that described above; yield, 50% of 2-(4'-methoxyphenyl)-propionic acid, m.p. 57°C.

Example 2

Preparation of 2-(2-thienyl)-propionic acid:

a) Dibenzoylperoxide (25.5 g; 0.107 mole) and iodine (14.9; 0.0588 mole) were added to a solution of 2-propionylthiophen (15 g; 0.107 mole) in methanol (10 g) and trimethylorthoformate (40 g; 0,377 mole) containing hydrochloric acid gas (0.17 g) and maintained under stirring for 3 hours at 20°C. The mixture was allowed to stand at room temperature for 1 hour, then heated to 70°C for 12 hours. After cooling to 30°C, sodium sulfite (5 g) and water (5 ml) were added and the reaction mixture was stirred for 30 minutes. Sodium hydroxide drops (8.8 g; 0.23 mole) were then added. The mixture was refluxed for 1 hour, acidified with 37% hydrochloric acid to pH 2—3, and extracted with ethyl ether. The extracts were dried, and the solvent and the product were distilled. 5.0 g of the desired product were obtained, b.p. 130°C (3.5 mmHg); yield, 30%. Thgis product is an useful intermediate for preparing Tiaprofenic acid.

Working in a similar manner, dl 2-(6'-methoxy-2'-naphthyl)propionic acid was prepared by substituting the 2-propionylthiophen with 1-(6'-methoxy-2'-naphthyl)-propanone and the dibenzoylperoxide with:
laurylperoxide (reaction time: 12 h; reaction temperature: 50°C); yield, 80%;
tert-butylperacetate (reaction time: 15 h; reaction temperature: 50°C); yield, 91%;
tert-butylhydroperoxide (reaction time: 12 h; reaction temperature: 50°C); yield, 70%;
(4-tert-butylcyclohexyl)-percarbonate (reaction time; 40 h; reaction temperature: 40°C); yield, 85%.

b) To a solution of 2-propionylthiophen (5 g; 35.7 mmol), methanol (5 g) and trimethylorthoformate (10 g; 94.2 mmole), were added dibenzoylperoxide (8.6 g; 35.7 mmole) and iodine (4.98 g; 19.6 mmole) portionwise is 1/2 hour. The mixture was heated to 40°C for 4 hours and 60°C for 15 hours. Then anhydrous sodium sulfite (1.5 g) and water (3 ml) were added, the mixture was stirred for 1/2 hour, sodium hydroxide (3.5 g; 88 mole) was added and the mixture was refluxed for 2 hours. After distillation under reduced pressure at 50°C, the residue was taken up with water (100 ml) and the thus obtained mixture was extracted with methylene chloride (2 × 10 ml), discarding the organic solvent. The aqueous layer was acidified with 37% hydrochloric acid to pH 2—3 and extracted with methylene chloride (3 × 20 ml). The organic extracts were dried and the solvent was removed by distillation.

4.0 g of the desired product were obtained thus (titre, 97.5%); yield, 70%.

Working in a similar manner but substituting the 2-propionyltiophen with:
1-(6'-methoxy-2'-naththyl)-propanone (reaction time: 17 hours;
reaction temperature: 50°C), the yield of 2-(6'-methoxy-2'-naphthyl)-propionic acid was 90%;
1-(4'-isobutyl-phenyl)-propane (reaction time: 17 hours;
reaction temperature: 60°C), the yield of 2-(4'-isobutylphenyl)propionic acid was 28%; m.p. 76°C;
propionylphenyl (reaction time: 28 hours; reaction temperature: 70°C), the yield of 2-phenyl-propionic acid was 32%;
1-(3'-phenoxy-phenyl)-propanone (reaction time: 32 hours; reaction temperature: 70°C), the yield of α-dl-2-(3-phenoxy-phenyl)-propionic acid was 30%.

Similarly but adding slowly 1.05 moles of iodine for each mole of ketone and without adding the oxidant
— 2-propionylthiophen yielded 70% of 2-(2-thienyl)-propionic acid; and
1-(4'-isobutyl-phenyl)-propanone yielded 53% of 2-(4'-isobutylphenyl)-propionic acid.

Example 3

Iodine (28 g; 0.11 mole) was added to a solution of 1-(6'-methoxy-2'-naphthyl)propanone) 21.4 g; 0.1 mole) in triethylorthoformate (120 ml; 0.72 mole) at room temperature. The mixture was stirred for 4 hours and then heated to reflux (67°C) for 24 hours. The low boiling compounds were distilled up to 85°C. The mixture was cooled to 50°C and anhydrous sodium sulfite (1 g) and deionized water (2 ml) were added. The mixture was stirred at the same temperature for 15 minutes and then sodium hydroxide (4.4 g; 0.11 mole) was added. The mixture was refluxed for 1 hour, diluted with water (500 ml), and acidified with 37% hydrochloric acid to pH 1. The precipitate was filtered washing with water to neutrality and dried to 70°C for 12 hours under reduced pressure.

18.4 g (titer 98%) of the desired product were obtained in this manner; yield, 79%.

Similar results were obtaind by working in a similar manner but substituting the triethylorthoformate with $C(OCH_3)_4$; Yield, 87%;
triisopropylorthoformate and isopropyl alcohol; Yield, 81%.

**Claims**

1. Process for preparing an arylalkanoic acid of formula

$$Ar{-}\overset{\displaystyle X}{\overset{|}{C}H}{-}COOH \qquad (II)$$

wherein X is H or a $C_1$—$C_4$ alkyl radical, and Ar is selected from the group comprising an aryl, a substituted aryl, a fused heterocyclic aryl, a heterocycle, a substituted heterocycle and a fused aryl heterocycle radical; characterized in that iodine is added to a mixture of an arylalkanone of the formula

$$Ar{-}CO{-}CH_2X \qquad (I)$$

wherein Ar and X have the meanings indicated above, and an excess of an orthoester, the reaction mixture is heated, then an inorganic base and finally an acid is added.

2. Process according to claim 1, characterized in that 1 mole of an arylalkanone of formula (I) is reacted with 0.5—1.5 mole of iodine and at least 2 mole of an orthoester.

3. Process according to any one of claims 1 and 2, characterized in that an oxidant is added.

4. Process according to one or more of claims 1 to 3, characterized in that an inert diluent is added.

5. Process according to one or more of claims 1 to 4, characterized in that a solvent is added.

6. Process according to one or more of claims 1 to 5, characterized in that a catalytic quantity of a protic acid is added.

7. Process according to claim 3, characterized in that the oxidant is hydrogen peroxide, an organic peroxide, a perester, a peracid or a hydroperoxide.

8. Process according to claim 7, characterized in that the organic peroxide is a diacylperoxide.

9. Process according to claim 8, characterized in that the diacylperoxide is dibenzoyl or dodecanoylperoxide.

10. Process according to claim 7, characterized in that the perester is di-tert-butyl-peracetate, tert-cyclohexylpercarbonate, tert-butylperbenzoate, di-tert-butylperoxalate, or di-tert-butyl-perisobutyrrate.

11. Process according to claim 7, characterized in that the peracid is m-chloroperbenzoic, or permaleic acid.

12. Process according to claim 7, characterized in that the hydroperoxide is tert-butylhydroperoxide, or cumylhydroperoxide.

13. Process according to claim 4, characterized in that the inert diluent is an aromatic hydrocarbon.

14. Process according to claim 13, characterized in that the aromatic hydrocarbon is benzene, toluene, xylene or nitrobenzene..

15. Process according to one or more of claims 1 to 14, characterized in that the orthoester is an alkylorthoformate, an alkylorthoacetate or an alkylorthocarbonate in which the alkyl radical has from 1 to 4 carbon atoms.

16. Process according to one or more of claims 1 to 15, characterized in that X is hydrogen or methyl and Ar is 6-methoxy-2-naphthyl, 2-thienyl, 4-isobutylphenyl, or 3-phenoxyphenyl.

**Patentansprüche**

1. Verfahren zur Herstellung einer Arylalkancarbonsäure der Formel

$$Ar{-}\overset{\displaystyle X}{\overset{|}{C}H}{-}COOH \qquad (II)$$

worin X für H oder einen Cl-C4-Alkylrest steht, und Ar ausgewählt ist unter einem Arylrest, einem substituierten Arylrest, einem kondensierten heterocyclischen Arylrest, einem heterocyclischen Rest, einem substituierten heterocyclischen Rest und einem kondensierten arylheterocyclischen Rest, dadurch gekennzeichnet, daß Jod zu einer Mischung aus einem Arylalkanon der Formel,

$$Ar{-}CO{-}CH_2X \qquad (I)$$

worin Ar und X die oben angegebenen Bedeutungen haben, und einem Überschuß eines Orthoesters gegeben wird, das Reaktionsgemisch erhitzt wird, dann eine anorganische Base und schlkießlich eine Säure hinzugefügt wird.

2. Verfahren nach Anspruch 1, dadurch gekennzeichnet, daß 1 Mol eines Arylalkanons der Formel (I) umgesetzt wird mit 0,5—1,5 Mol Jod und mit mindestens 2 Mol des Orthoesters.

3. Verfahren nach einem der Ansprüche 1 und 2, dadurch gekennzeichnet, daß ein Oxidationsmittel hinzugefügt wird.

4. Verfahren nach einem oder mehreren der Ansprüche 1 bis 3, dadurch gekennzeichnet, daß ein inertes Verdünnungsmittel hinzugefügt wird.

5. Verfahren nach einem oder mehreren der Ansprüche 1 bis 4, dadurch gekennzeichnet, daß ein Lösungsmittel hinzugefügt wird.

6. Verfahren nach einem oder mehreren der Ansprüche 1 bis 5, dadurch gekennzeichnet, daß eine katalytische Menge einer protischen Säure hinzugefügt wird.

7. Verfahren nach Anspruch 3, dadurch gekennzeichnet, daß das Oxidationsmittel Wasserstoffperoxid, ein organisches Peroxid, ein Perester, eine Persäure oder ein Hydroperoxid ist.

8. Verfahren nach Anspruch 7, dadurch gekennzeichnet, daß das organische Peroxid ein Diacylperoxid ist.

9. Verfahren nach Anspruch 8, dadurch gekennzeichnet, daß das Diacylperoxid Dibenzoyl oder dodecanoylperoxid ist.

10. Verfahren nach Anspruch 7, dadurch gekennzeichnet, daß der Perester Di-t-butylperacetat, t-cyclohexylpercarbonat, t-Butylperbenzoat, Di-t-butylperoxalat oder Di-t-butylperisobutyrat ist.

11. Verfahren nach Anspruch 7, dadurch gekennzeichnet, daß die Persäure m-Perchlorbenzoesäure oder Permaleinsäure ist.

12. Verfahren nach Anspruch 7, dadurch gekennzeichnet, daß das Hydroperoxid t-Butylhydroperoxid oder Cumylhydroperoxid ist.

13. Verfahren nach Anspruch 4, dadurch gekennzeichnet, daß das inerte Verdünnungsmittel ein aromatischer Kohlenwasserstoff ist.

14. Verfahren nach Anspruch 13, dadurch gekennzeichnet, daß der aromatische Kohlenwassestoff Benzol, Toluol, Xylol oder Nitrobenzol ist.

15. Verfahren nach einem oder mehreren der Ansprüche 1 bis 14, dadurch gekennzeichnet, daß der Orthoester ein Alkylorthoameisensäureester, ein Alkylorthoacetat oder ein Alkylorthocarbonat ist, wobei der Alkylrest 1 bis 4 Kohlenstoffatome aufweist.

16. Verfahren nach einem oder Mehreren der Ansprüche 1 bis 15, dadurch gekennzeichnet, daß X Wasserstoff oder eine Methylgruppe bezeichnet und Ar ein 6-Methoxy-2-naphthyl-, 2-Thienyl-, 4-Isobutylphenyl- oder 3-Phenoxyphenylrest ist.

**Revendications**

1. Procédé pour préparer un acide arylalcanoïque de formule:

$$Ar—\underset{}{\overset{X}{\overset{|}{C}}}H—COOH \qquad (II)$$

dans laquelle X est H ou un radical alkyle en $C_1$—$C_4$, et Ar est choisi parmi un radical alkyle, un radical aryle substitué, un radical aryle hétérocyclique condensé, un hétérocycle, un hétérocycle substitué et un radical aryl-hétérocycle condensé; caractérisé en ce que l'on ajoute de l'iode à un mélange d'une arylalcanone de formule:

$$Ar—CO—CH_2X \qquad (I)$$

dans laquelle Ar et X ont les significations indiquées ci-dessus, et d'un excès d'une orthoester, on chauffe le mélange de réaction, ensuite on ajoute une base inorganique et enfin un acide.

2. Procédé selon la revendication 1, caractérisé en ce que l'on fait réagir 1 mole d'une arylalcanone de formule (I) avec 0,5—1,5 mole d'iode et au moins 2 moles d'un orthoester.

3. Procédé selon l'une quelconque des revendications 1 et 2, caractérisé en ce que l'on ajoute un oxydant.

4. Procédé selon une ou plusieurs des revendications 1 à 3, caractérisé en ce que l'on ajoute un dilant inerte.

5. Procédé selon une plusieurs des revendications 1 à 4, caractérisé en ce que l'on ajoute un solvant.

6. Procédé selon une ou plusieurs des revendications 1 à 5, caractérisé en ce que l'on ajoute une quantité catalytique d'un acide protique.

7. Procédé selon la revendication 3, caractérisé en ce que l'oxydant est le peroxyde d'hydrogène, un peroxyde organique, un perester, un peracide ou un hydroperoxyde.

8. Procédé selon la revendication 7, caractérisé en ce que le peroxyde organique est un peroxyde de diacyle.

9. Procédé selon la revendication 8, caractérisé en ce que le peroxyde de diacyle est le peroxyde de dibenzoyle ou de dedécanoyle.

10. Procédé selon la revendication 7, caractérisé en ce que le perester est le peracétate de di-tert-butyle,

le percarbonate de tert-cyclohexyle, le perbenzoate de tert-butyle, le peroxalate de di-tert-butyle ou le perisobutyrate de di-tertbutyle.

11. Procédé selon la revendication 7, caractérisé en ce que le peracide est l'acide m-chloroperbenzoïque ou l'acide permaléique.

12. Procédé selon la revendication 7, caractérisé en ce que l'hydroperoxyde est l'hydroperoxyde de tert-butyle ou l'hydroperoxyde de cumyle.

13. Procédé selon la revendication 4, caractérisé en ce que le diluant inerte est un hydrocarbure aromatique.

14. Procédé selon la revendication 13, caractérisé en ce que l'hydrocarbure aromatique est le benzène, le toluène, le xylène ou le nitrobenzène.

15. Procédé selon l'une ou plusieurs des revendications 1 à 14, caractérisé en ce que l'orthoester est un orthoformiate d'alkyle, un orthoacétate d'alkyle ou un orthocarbonate d'alkyle dans lequel le radical alkyle a de 1 à 4 atomes de carbone.

16. Procédé selon une ou plusieurs des revendications 1 à 15, caractérisé en ce que X est l'hydrogène ou un radical méthyle et Ar est un radical 6-méthoxy-2-naphtyle, 2-thiényle, 4-isobutylphényle ou 3-phénoxyphényle.